(19) Europäisches Patentamt
European Patent Office
Office européen des brevets

(11) Veröffentlichungsnummer: **0 072 988**
**B1**

(12) # EUROPÄISCHE PATENTSCHRIFT

(45) Veröffentlichungstag der Patentschrift: **02.10.85**

(51) Int. Cl.⁴: **C 07 C 13/18**, C 07 C 1/253, C 07 C 1/22, A 61 K 7/00

(21) Anmeldenummer: **82107362.4**

(22) Anmeldetag: **13.08.82**

(54) **Verwendung von 1,3-Dialkyl-cyclohexan-Verbindungen als kosmetische Ölkomponente.**

(30) Priorität: **21.08.81 DE 3133078**

(43) Veröffentlichungstag der Anmeldung: **02.03.83 Patentblatt 83/9**

(45) Bekanntmachung des Hinweises auf die Patenterteilung: **02.10.85 Patentblatt 85/40**

(84) Benannte Vertragsstaaten: **AT BE CH DE FR GB IT LI NL**

(56) Entgegenhaltungen:
**US - A - 3 748 231**

**CHEMICAL ABSTRACTS SERVICE REGISTRY HANDBOOK, Number Section, 1975 supplement, American Chemical Society, Columbus, USA**
**HOUBEN-WEYL: "Methoden der organischen Chemie", 4. Auflage, Band V/1a, Kohlenwasserstoffe, Teil 1, 1970, Georg Thieme Verlag, Stuttgart, DE.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Conrad, Jens, Dr., Dürerweg 15, D-4010 Hilden (DE)**
Erfinder: **Schaper, Ulf-Armin, Dr., Nixenstrasse 17, D-4000 Düsseldorf 13 (DE)**
Erfinder: **Zeidler, Ulrich, Dr., Heinrich-Lersch-Strasse 19, D-4000 Düsseldorf 13 (DE)**

**EP 0 072 988 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft die Verwendung von 1,3-Dialkyl-substituierten Cyclohexan-Verbindungen als kosmetische Ölkomponente sowie diese Verbindungen enthaltende Mittel zur Pflege und Behandlung des Gesichts, des Körpers oder der Frisur.

1,3-Didecylcyclohexan ist aus Chemical Abstracts Service Registry Handbook, Number Section, 1975 Supplement, American Chemical Society Seite 995 RD, Nr. 55 334-19-5 bekannt. 1,3-Di-n-propylcyclohexan wird in US-A-3 748 231 erwähnt. Die Hydrierung von 2-Oxo-1,3-dimethylcyclohexan zu 1,3-Dimethylcyclohexan wird in Houben-Weyl »Methoden der organischen Chemie«, 4. Auflage (1970), Band V/1a, Seite 269 beschrieben.

2,6-Dialkylcyclohexanole und Verfahren zu deren Herstellung sind in Fette, Seifen, Anstrichmittel, (1980), Band 82, Seiten 454—456 beschrieben. Davon ausgehend wurden die 1,3-Dialkylcyclohexan-Verbindungen hergestellt und es wurde überraschend festgestellt, daß diese besonders gute kosmetische Ölkomponenten darstellen, welche sich ausgezeichnet zum Beispiel zu farblosen Emulsionen und glatten Cremes für kosmetische Zwecke verarbeiten lassen.

Aufgabe der vorliegenden Erfindung ist es, für die Verwendung in kosmetischen Mitteln geeignete Ölkomponenten bereitzustellen.

Die geeigneten Verbindungen sind durch die folgende allgemeine Formel (I)

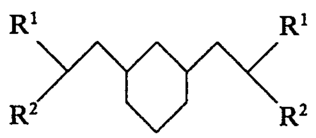

(I)

worin $R^1$ für Wasserstoff, einen linearen oder einen verzweigten $C_1-C_{20}$-Alkylrest und $R^2$ für einen linearen oder verzweigten $C_1-C_{20}$-Alkylrest stehen, darstellbar. Bevorzugt werden Verbindungen in denen $R^1$ und $R^2$, welche gleich oder verschieden sein können, einen linearen Alkylrest darstellen, vor allem solche Verbindungen, in denen $R^1$ und $R^2$ für Methyl, Ethyl, n-Propyl, n-Butyl, n-Pentyl oder n-Hexyl stehen.

Diese Verbindungen werden nach einem Verfahren gemäß folgender Reaktionsgleichung dargestellt:

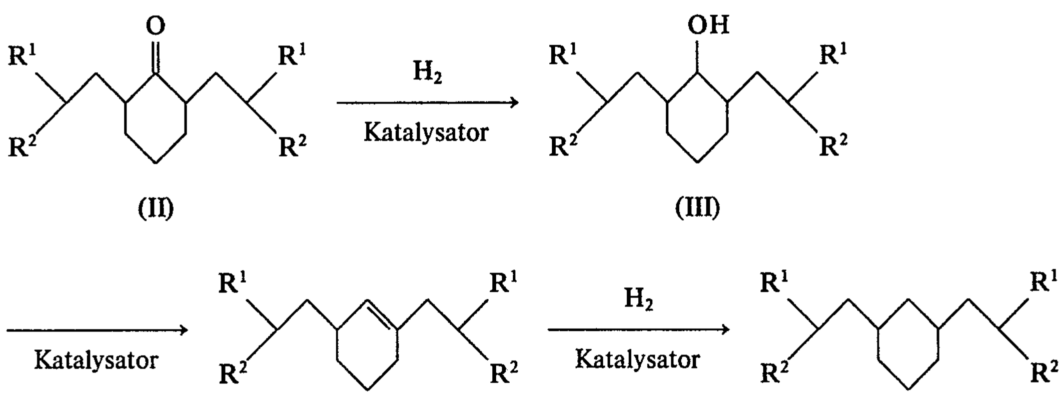


(II) (III) (IV) (I)

wobei $R^1$ und $R^2$ die obengenannte Bedeutung haben. Als Ausgangspunkt für die Herstellung der 1,3-Dialkylcyclohexane verwendet man ein entsprechend 2,6-Dialkyl-substituiertes Cyclohexanon (II) oder Cyclohexanol (III), welches unter hydrierenden Bedingungen in Gegenwart eines Katalysators bei erhöhter Temperatur und einem Wasserstoffdruck von 10—300 bar einige Stunden, zum Beispiel 5 bis 15 Stunden, hydriert wird. Alle Reaktionsschritte werden ohne Isolierung der jeweiligen Zwischenprodukte im Eintopfverfahren durchgeführt. Als Hydrierkatalysator eignen sich zum Beispiel metallisches Nickel, Kobalt, Eisen, Eisen oder Edelmetalle wie Platin oder Palladium. Bevorzugt wird die Reaktion mit einem Nickelkatalysator bei Temperaturen von 200 bis 300° C ausgeführt. Die als Ausgangsprodukte eingesetzten 2,6-Dialkylcyclohexanol-Verbindungen werden durch eine gemischte Aldolkondensation zwischen 2 Mol Aldehyd der Formel V

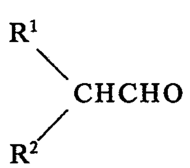

(V)

worin $R^1$ und $R^2$ die obengenannte Bedeutung haben und einem Mol Cyclohexanon und nachfolgender Hydrierung erhalten. Alternativ können die 2,6-Dialkylcyclohexanol-Verbindungen auch aus Alkoholen der Formel VI

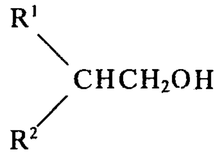

(VI)

wobei $R^1$ und $R^2$ die obengenannte Bedeutung haben, und Cyclohexanol unter den Bedingungen der gemischten Guerbet-Reaktion erhalten werden. Dieses Verfahren zur Herstellung von 2,6-Dialkylcyclohexanol ist in Fette, Seifen, Anstrichmittel (1980) Band 82 (Seiten 554—556) beschrieben.

Die 1,3-Dialkylcyclohexan-Verbindungen liegen in Form von farblosen Ölen vor. Sie sind geruchlos und sie eignen sich besonders gut als kosmetische Ölkomponenten. Sie haben rückfettende Eigenschaften, und sie sind sehr gut hautverträglich. Die bisher in kosmetischen Cremes als Ölkomponente und Salbengrundlage verwendeten gesättigten, verzweigten Kohlenwasserstoffe wie Vaseline, führen auf der Haut zu einem Wärmestau, da sie die Poren der Haut verstopfen. Dieser Wärmestau bewirkt ein zumeist als unangenehm empfundenes heißes Gefühl. Dieser unerwünschte Effekt wird bei der erfindungsgemäßen Verwendung der 1,3-Dialkylcyclohexan-Verbindungen als Ölkomponente vermieden. Als besonders vielseitig verwendbar hat sich 1,3-Di-(2-ethylhexyl)-cyclohexan erwiesen. Die 1,3-Dialkylcyclohexan-Verbindungen lassen sich in verschiedene kosmetische Zubereitungen, wie in Suspensionen, Gelen, Emulsionen, Salben, Pasten oder Schüttelmixturen gut einarbeiten. Als kosmetische Mittel, in denen die Verbindungen verwendbar sind, kann man zum Beispiel Hautcremes, Körpermilch, Reinigungsmilch, Schminkgrundlagen, Sonnenschutzmittel, Haarpflegemittel oder Badepräparate nennen.

Neben den 1,3-Dialkylcyclohexan-Verbindungen können die erfindungsgemäßen kosmetischen Mittel andere übliche Bestandteile und Hilfsmittel, wie z. B. Konservierungsmittel, Sequestriermittel, Parfüms, Lösungsmittel, Eintrübungsmittel, Verdickungsmittel, Farbstoffe, pH-modifizierende Zusätze, Pflanzenextrakte, hautpflegende Wirkstoffe wie Kollagene, Fettsäuren, Fettsäureester, Glykole, Glykolether, tierische, pflanzliche oder synthetische Öle enthalten.

In der erfindungsgemäßen kosmetischen Mitteln liegen die 1,3-Dialkylcyclohexan-Verbindungen in Mengen von 2 bis 25 Gew.-%, vorzugsweise 5 bis 15 Gew.-%, bezogen auf das Gesamtgewicht des kosmetischen Mittels, vor.

Die folgenden Beispiele sollen die vorliegende Erfindung näher erläutern:

## Beispiele

1. Herstellung von 1,3-Di-(2-ethylhexy)-cyclohexan

300 g 2,6-Di(2-ethylhexyl)-cyclohexanol wurden mit 30 g Girdler Nickel 49A sechs Stunden bei 250° C und 250 bar Wasserstoffdruck hydriert. Nach dem Abfiltrieren des Katalysators verblieben 260 g 1,3-Di-(2-ethylhexyl)-cyclohexan mit folgenden Kennzahlen:

| | |
|---|---|
| Säurezahl: | 0,1 |
| Hydroxyzahl: | 0 |
| Jodzahl: | 0 |
| Molmasse: | 308 (Theorie 308) |
| IR (Öl) $cm^{-1}$: | 2965, 2930, 2880, 2865, 1460, 1380 |

2. Herstellung von 1,3-Di-(2-ethylhexyl)-cyclohexan

200 g 2,6-Di-(2-ethylhexyl)-cyclohexanol wurden mit 10 g eines Nickelkatalysators, der ca. 22% Nickel auf Kieselgur in einem Hartfett gebunden, enthält (Pricat 9904 der Fa. Unichema), 13 Stunden bei 250° C und 20 bar Wasserstoffdruck hydriert. Nach dem Abfiltrieren des Katalysators wurden 171 g 1,3-Di-(2-ethylhexyl)-cyclohexan durch Destillation bei 0,01 mbar zwischen 118° und 123° erhalten.

Kennzahlen:

| | |
|---|---|
| Säurezahl: | 0,2 |
| Hydroxylzahl: | 1,5 |
| Jodzahl: | 1,4 |
| IR (Öl) $cm^{-1}$: | 2965, 2930, 2880, 2865, 1460, 1380. |

3. Dieselbe Reaktion wurde mit 20 g Pricatkatalysator wiederholt. Nach 15 Stunden Hydrierzeit wurde 160 g Endprodukt mit folgenden Kennzahlen erhalten:

| | |
|---|---|
| Säurezahl: | 0,1 |
| Jodzahl: | 0,4 |
| Hydroxylzahl: | 0 |
| $Kp_{0,013}$: | 127° C |

4. Herstellung von 1,3-Diisobutylcyclohexan

85 g 2,6-Diisobutylcyclohexanol wurden mit 8,5 g Girdler-Nickel 49A 8 Stunden bei 250° C und 250 bar Wasserstoffdruck hydriert. Nach dem Abfiltrieren des Katalysators wurden durch Destillation 61 g des Kohlenwasserstoffes isoliert.

| | |
|---|---|
| $Kp_{0,07}$ | 51° C |
| Säurezahl, SZ: | 0,1 |
| Hydroxylzahl, OHZ: | 0 |
| Molmasse: | 196 |
| Theorie: | 196 |
| IR (Öl) $cm^{-1}$: | 2960, 2930, 2865, 2845, 1470, 1385, 1370, 1170 |

5. 1,3-Di(2-ethylbutyl)cyclohexan

Wie im Beispiel 4 beschrieben, wurden aus 94 g 2,6-Di(2-ethylbutyl)cyclohexanol mit 9,4 g Girdler-Nickel 49A 62 g Kohlenwasserstoff hergestellt.

| | |
|---|---|
| $Kp_{0,01}$ | 93—95° C |
| SZ: | 0 |
| OHZ: | 0,5 |
| Molmasse: | 252 |
| Theorie | 252 |
| IR (Öl) $cm^{-1}$: | 2960, 2920, 2865, 2860, 1460, 1380 |

6. 1,3-Di(2-methylpentyl)cyclohexan

Wie im Beispiel 4 beschrieben, wurden aus 100 g 2,6-Di(2-methylpentyl)-cyclohexanol mit 10 g Girdler-Nickel 49A 75 g Kohlenwasserstoff hergestellt.

| | |
|---|---|
| $Kp_{0,01}$: | 88—91° C |
| SZ: | 0 |
| OHZ: | 0 |
| Molmasse: | 252 |
| Theorie: | 252 |
| IR (Öl) $cm^{-1}$: | 2965, 2930, 2870, 2850, 1468, 1455, 1380,1155 |

7. 1,3-Didecylcyclohexan

137 g 2,6-Didecylcyclohexanol wurden mit 14 g Girdler-Nickel 49A zu 71 g Kohlenwasserstoff hydriert.

| | |
|---|---|
| $Kp_{0,01}$: | 174—180° C |
| SZ: | 0 |
| OHZ: | 0 |
| Molmasse: | 364 |
| Theorie: | 364 |
| IR (Öl) $cm^{-1}$: | 2965, 2925, 2860, 1468, 1460, 1380 |

Nachfolgend werden Beispiele für erfindungsgemäße kosmetische Mittel angegeben:

8. Flüssige O/W-Emulsion

| | | |
|---|---|---|
| I | Lanette O (Fettalkohol) | 3,5% |
| | Eumulgin B 1 | 3,0% |
| | 1,3-Di-(2-ethylhexyl)-cyclohexan | 5,0% |
| II | 1,2-Propylenglykol | 3,0% |
| | Harnstoff | 2,0% |
| | Wasser | 83,5% |

9. O/W-Creme

| | | |
|---|---|---|
| I | Eumulgin B 3 (Fettalkoholpolyglykolether) | 13,0% |
| | Cetiol HE (Fettsäureester) | 20,0% |
| | 1,3-Di-(2-ethylhexyl)-cyclohexan | 5,0% |
| II | Clycerin 86%ig | 20,0% |
| | Wasser | 42,0% |

10. Frisiercreme O/W

| | | |
|---|---|---|
| I | Eumulgin B 1 (Cetylstearylalkohol mit ca. 12 Mol Ethylenoxid) | 5,0% |
| | Cutina MD (Fettsäureglyceridgemisch) | 15,0% |
| | 1,3-Di-(2-ethylhexyl)-cyclohexan | 20,0% |
| II | Wasser | 60,0% |

11. Reinigungscreme O/W

| | | |
|---|---|---|
| I | Lanette 16 (Cetylalkohol) | 2,0% |
| | Cutina MD (Fettsäureglyceridgemische) | 14,0% |
| | Eumulgin B 1 (Cetylstearylalkohol mit ca. 12 Mol Ethylenoxid) | 1,5% |
| | Eumulgin B 2 (Fettalkoholpolyglykolether) | 1,5% |
| | Cetiol LC (Fettsäureester) | 7,0% |
| | 1,3-Di-(2-ethylhexyl)-cyclohexan | 15,0% |
| II | Wasser | 59,0% |

12. W/O-Creme

| | | |
|---|---|---|
| I | Dehymuls K (Emulgator) | 25,0% |
| | Myritol 318 (Vorlauffettsäureglycerinester) | 10,0% |
| | 1,3-Di-(2-ethylhexyl)-cyclohexan | 5,0% |
| II | Wasser | 60,0% |

13. W/O-Creme

| | | |
|---|---|---|
| I | Dehymuls F (Emulgator) | 8,0% |
| | Vaseline, weiß | 15,0% |
| | 1,3-Di-(2-ethylhexyl)-cyclohexan | 8,0% |
| II | Clycerin 87%ig | 3,0% |
| | $MgSO_4 \cdot 7\ H_2O$ | 0,3% |
| | Wasser | 65,7% |

14. W/O-Creme

| | | |
|---|---|---|
| I | Dehymuls F (Emulgator) | 7,0% |
| | Cetiol V (Ölsäuredecylester) | 6,0% |
| | Bienenwachs, weiß | 3,0% |
| | Vaseline, weiß | 12,0% |
| | 1,3-Diisobutylcyclohexan | 6,0% |
| II | Glycerin 86%ig | 5,0% |
| | $MgSO_4 \cdot 7\ H_2O$ | 0,3% |
| | Wasser | 60,7% |

**Patentansprüche**

1. Die Verwendung von 1,3-Dialkylcyclohexan-Verbindungen der allgemeinen Formel I

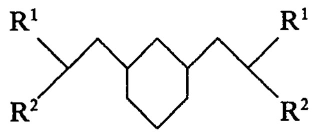

(I)

worin $R^1$ für Wasserstoff, einen geradkettigen oder einen verzweigten $C_1-C_{20}$-Alkylrest und $R^2$ für einen geradkettigen oder verzweigten $C_1-C_{20}$-Alkylrest stehen, als kosmetische Ölkomponente.

2. Die Verwendung nach Anspruch 1, dadurch gekennzeichnet, daß $R^1$ und $R^2$ für einen $C_1-C_6$-Alkylrest stehen.

3. Die Verwendung nach Anspruch 1, dadurch gekennzeichnet, daß als 1,3-Dialkylcyclohexan-Verbindung das 1,3-Di-(2-ethylhexyl)-cyclohexan verwendet wird.

4. Mittel für kosmetische Zwecke, dadurch gekennzeichnet, daß sie als kosmetische Ölkomponente 3 bis 25 Gewichtsprozent 1,3-Dialkylcyclohexan-Verbindung der Formel I nach Anspruch 1, bezogen auf das Gewicht des gesamten Mittels, neben üblichen kosmetischen Bestandteilen und Hilfsmitteln enthalten.

**Claims**

1. The use of 1,3-dicyclohexane compounds corresponding to the following general formula

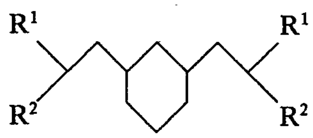

(I)

in which $R^1$ represents hydrogen or a linear or branched $C_1-C_{20}$ alkyl radical and $R^2$ represents a linear or branched $C_1-C_{20}$ alkyl radical, as a cosmetic oil component.

2. The use claimed in Claim 1, characterized in that $R^1$ and $R^2$ represent a $C_1-C_6$ alkyl radical.

3. The use claimed in Claim 1, characterized in that 1,3-di-(2-ethylhexyl)-cyclohexane is used as the 1,3-dialkyl cyclohexane compound.

4. Preparations for cosmetic purposes, characterized in that they contain as cosmetic oil component from 3 to 25% by weight of the 1,3-dialkylcyclohexane compound corresponding to formula I in Claim I, based on the weight of the preparation as a whole, in addition to standard cosmetic constituents and auxiliaries.

**Revendications**

1. Utilisation de composés de 1,3-dialcoylcyclohexane de formule générale I

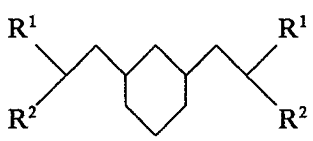

(I)

dans laquelle $R^1$ représente de l'hydrogène, un radical alcoyle en $C_1-C_{20}$ à chaîne droite ou ramifiée et $R^2$ un radical alcoyle en $C_1-C_{20}$ à chaîne droite ou ramifiée, en tant que composants huileux cosmétiques.

2. Utilisation selon la revendication 1, caractérisée en ce que $R^1$ et $R^2$ représentent un radical alcoyle en $C_1-C_6$.

3. Utilisation selon la revendication 1, caractésrisée en ce qu'on utilise comme composé de 1,3-dialcoylcohexane le 1,3-di-(2-éthylhexyl)-cyclohexane.

4. Agents pour applications cosmétiques, caractérisés en ce qu'ils contiennent comme composant huileux cosmétique 3 à 25% en poids d'un composé de 1,3-dialcoylcyclohexane de formule I selon la revendication 1, par rapport au poids de l'agent total, à côté de constituants et d'auxiliaires cosmétiques courants.